(19) 

Europäisches
Patentamt
European
Patent Office
Office européen
des brevets

(11)  **EP 4 231 000 A1**

(12)  **EUROPEAN PATENT APPLICATION**
published in accordance with Art. 153(4) EPC

(43) Date of publication:
**23.08.2023  Bulletin 2023/34**

(21) Application number: **21880105.8**

(22) Date of filing: **12.10.2021**

(51) International Patent Classification (IPC):
**G01N 21/359** (2014.01)

(52) Cooperative Patent Classification (CPC):
**G01N 21/359**

(86) International application number:
**PCT/JP2021/037735**

(87) International publication number:
**WO 2022/080367 (21.04.2022 Gazette 2022/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **13.10.2020   JP 2020172722**

(71) Applicant: **Nippon Shokubai Co., Ltd.
Osaka-shi, Osaka 541-0043 (JP)**

(72) Inventors:
• **NODA, Atsuhiro
  Himeji-shi, Hyogo 671-1282 (JP)**
• **HORIE, Kazushi
  Suita-shi, Osaka 564-0034 (JP)**
• **NAKATANI, Kaori
  Suita-shi, Osaka 564-0034 (JP)**

(74) Representative: **Hoffmann Eitle
Patent- und Rechtsanwälte PartmbB
Arabellastraße 30
81925 München (DE)**

(54)  **PREDICTION METHOD AND PREDICTION DEVICE**

(57)  In a process for producing a resin powder, a physical property of a water absorbent resin powder is predicted from a near-infrared absorption spectrum. A predicting apparatus (100) includes: a measurement data obtaining section (11) which obtains near-infrared measurement data: and a predicting section (13) which inputs, into a prediction model, at least any one selected from the group consisting of the near-infrared measurement data and one or more pieces of processed data which have been generated on the basis of the near-infrared measurement data and outputs prediction information concerning a physical property of a resin powder.

EP 4 231 000 A1

FIG. 2

**Description**

Technical Field

The present disclosure relates to a predicting method and a predicting apparatus, each of which is for predicting a physical property of a water absorbent resin powder.

Background Art

A water absorbent resin (super absorbent polymer, hereinafter abbreviated to "SAP") is a resin having a water-swelling property and water-insolubility. The SAP is often in powder form (or particulate form). As the performance of the water absorbent resin, known are a fluid retention capacity (CRC), an absorption against pressure (AAP), a water absorption speed, a saline flow conductivity (SFC), and the like. Desired physical property values and the ranges of the physical property values vary depending on an intended use of the SAP, specifically, the type or configuration of a hygienic material in which the SAP is used. Therefore, a SAP which exhibits a wide variety of physical property values depending on the form of an end product is required.
In order to confirm physical properties of a SAP powder, it is necessary to apply different measurement methods to each physical property measurement item, and it takes a given time to carry out each measurement. In production of a SAP, since it is difficult to ascertain, in real time, physical property values of the SAP in each step, there is a risk of producing an off-spec product. That is, there is a risk of causing a decrease in yield in the production of the SAP.
Patent Literature 1 discloses a method for predicting a physical property of a water absorbent resin with use of a specific Raman spectrum measured with respect to the water absorbent resin.

Citation List

[Patent Literature]

[Patent Literature 1]
International Publication No. WO 2020/109601

Summary of Invention

Technical Problem

In measurement of a Raman spectrum, a target sample is irradiated with a specific single wavelength, and then scattered light with a specific range of wavenumbers is measured. Due to such characteristic of irradiation light, the Raman spectrum is unlikely to be affected by the particle diameter of a measurement target. Thus, the Raman spectrum is unsuitable for accurately measuring the particle diameter of a measurement target.
In contrast, in a method in which a near-infrared absorption spectrum is used, a target sample is irradiated with a plurality of wavelengths or a continuous spectrum of near-infrared light (generally, having a wavelength falling within a range of 750 nm to 2500 nm), and then transmitted, absorbed, refracted, reflected, and diffused light is measured. Therefore, it is possible to carry out measurement including information on the particle diameter of the target sample. Furthermore, in a case where near-infrared light is used, it is possible to carry out measurement with use of a wide wavelength range, and therefore possible to obtain more accurate average information on the target sample, as compared with a case where the Raman spectrum is measured.
An aspect of the present disclosure has an object to achieve predicting a physical property of a water absorbent resin powder from a near-infrared absorption spectrum in a process for producing the water absorbent resin powder.

Solution to Problem

In order to attain the above object, a predicting method according to an aspect of the present disclosure is a method for predicting a physical property of a resin powder (note: the resin powder indicates any one of a water absorbent resin powder and an intermediate product which is produced in a process for producing the water absorbent resin powder), the method including: a measurement data obtaining step of obtaining near-infrared measurement data which indicates a near-infrared absorption spectrum of the resin powder; and a predicting step of inputting, into a prediction model, at least one selected from the group consisting of the near-infrared measurement data and one or more pieces of processed data which have been generated on the basis of the near-infrared measurement data, and outputting prediction information concerning the physical property of the resin powder.

In order to attain the above object, a predicting apparatus according to an aspect of the present disclosure is a predicting apparatus which predicts a physical property of a resin powder (note: the resin powder indicates any one of a water absorbent resin powder and an intermediate product which is produced in a process for producing the water absorbent resin powder), the predicting apparatus including: a measurement data obtaining section (which obtains measurement data that indicates a near-infrared absorption spectrum measured with respect to the resin powder); and a predicting section (which inputs, into a prediction model, at least any one selected from the group consisting of the near-infrared measurement data and one or more pieces of processed data which have been generated on the basis of the near-infrared measurement data, and outputs prediction information concerning the physical property of the resin powder).

Advantageous Effects of Invention

An aspect of the present disclosure brings about the effect that it is possible to predict a physical property of a water absorbent resin powder from a near-infrared absorption spectrum.

Brief Description of Drawings

Fig. 1 is a block diagram illustrating an example of a configuration of a predicting system including a predicting apparatus according to Embodiment 1 of the present disclosure.
Fig. 2 is a functional block diagram illustrating an example of a configuration of a main part of the predicting apparatus.
Fig. 3 is a flowchart illustrating a flow of a process carried out by the predicting apparatus.
Fig. 4 is a functional block diagram illustrating an example of a configuration of a main part of the predicting apparatus which generates a prediction model.
Fig. 5 is a figure illustrating a data structure of near-infrared measurement data.
Fig. 6 is a figure illustrating a data structure of physical property information.
Fig. 7 is a flowchart illustrating a flow of a process carried out by the predicting apparatus which carries out machine learning.
Fig. 8 is a block diagram illustrating an example of a predicting system according to Embodiment 2 of the present disclosure.
Fig. 9 is a table showing correspondence between MAC addresses obtained by a predicting apparatus according to Embodiment 2 of the present disclosure and near-infrared spectrophotometers.
Fig. 10 is a graph showing correlation between actually measured values and predicted values of gel D50.
Fig. 11 is a graph showing correlation between actually measured values and predicted values of CRC.
Fig. 12 is a graph showing correlation between actually measured values and predicted values of AAP.
Fig. 13 is a graph showing correlation between actually measured values and predicted values of SFC.
Fig. 14 is a graph showing correlation between actually measured values and predicted values of D50.
Fig. 15 is a graph showing correlation between actually measured values and predicted values of a moisture content (solid content).

Description of Embodiments

Embodiment 1

The following description will discuss an embodiment of the present disclosure in detail.

(Configuration of predicting system 1000)

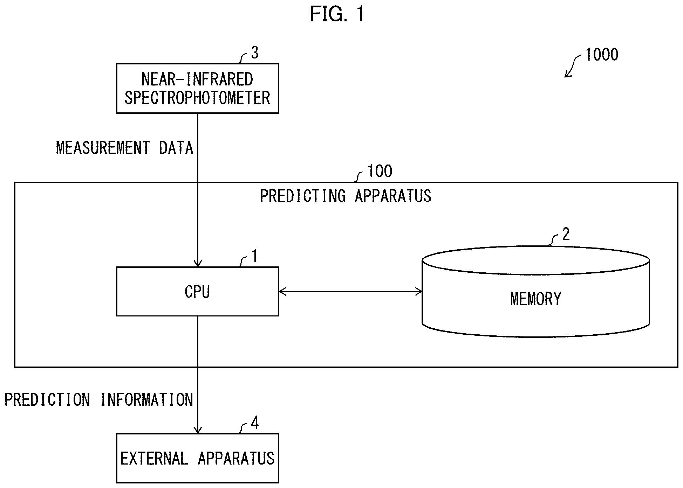

Firstly, a configuration of a predicting system 1000 including a predicting apparatus 100 according to an embodiment of the present disclosure will be described with reference to Fig. 1. Fig. 1 is a block diagram illustrating an example of the configuration of the predicting system 1000.
The predicting system 1000 includes the predicting apparatus 100, a near-infrared spectrophotometer 3, and an external apparatus 4.
The predicting apparatus 100 includes a CPU 1 and a memory 2. The predicting apparatus 100 may be communicably connected to the near-infrared spectrophotometer 3 and the external apparatus 4, as illustrated in Fig. 1. Communications between the predicting apparatus 100 and the near-infrared spectrophotometer 3 may be carried out via a network such as near field communication, wired connection, and the Internet. Alternatively, the communications between the predicting apparatus 100 and the near-infrared spectrophotometer 3 may be achieved by directly connecting the predicting apparatus 100 and the near-infrared spectrophotometer 3 with use of a connector such as a USB terminal. Communications between the predicting apparatus 100 and the external apparatus 4 are also similar to the communications between the predicting apparatus 100 and the near-infrared spectrophotometer 3.

Although Fig. 1 illustrates a case where a single near-infrared spectrophotometer 3 and a single external apparatus 4 are communicably connected to the predicting apparatus 100, the predicting system 1000 is not limited to such a configuration. A single or a plurality of near-infrared spectrophotometers 3 may be communicably connected to the predicting apparatus 100. Similarly, a single or a plurality of external apparatuses 4 may be communicably connected to the predicting apparatus 100.

The predicting apparatus 100 inputs, into a prediction model, at least any one selected from the group consisting of near-infrared measurement data which indicates a near-infrared absorption spectrum obtained from the near-infrared spectrophotometer 3 and one or more pieces of processed data which have been generated on the basis of the near-infrared measurement data, and outputs prediction information concerning a physical property of a resin powder. Herein, the "measurement data which indicates a near-infrared absorption spectrum" may be simply referred to as "near-infrared absorption spectrum (near-infrared measurement data)".

The near-infrared spectrophotometer 3 is an apparatus which measures light reflected by the resin powder and light transmitted by the resin powder when the resin powder is irradiated with near-infrared radiation and which calculates the near-infrared absorption spectrum that indicates a near-infrared radiation absorption characteristic of the resin powder. Note, here, that near-infrared radiation is light of which a wavelength range is 750 nm to 2500 nm. The near-infrared absorption spectrum will be described later.

The external apparatus 4 is any apparatus which receives a prediction result outputted from the predicting apparatus 100. As an example, the external apparatus 4 may be any display apparatus or may be a computer which is used by a manager who manages a process for producing the resin powder. Alternatively, the external apparatus 4 may be any production apparatus which carries out a process in the process for producing the resin powder.

(Configuration of predicting apparatus 100)

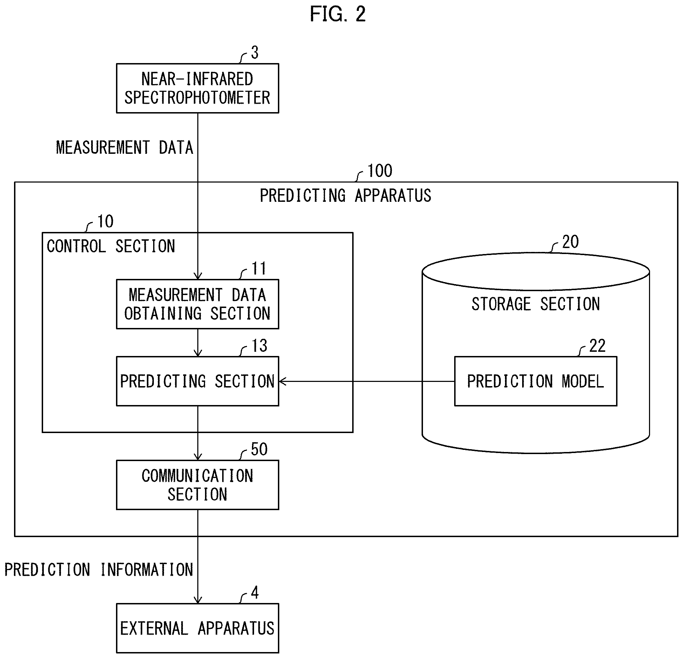

The following description will discuss a configuration of the predicting apparatus 100 which predicts a physical property of a water absorbent resin powder (hereinafter also simply referred to as "resin powder") with use of a prediction model 22, with reference to Fig. 2. Fig. 2 is a functional block diagram illustrating an example of a configuration of a main part of the predicting apparatus 100.

Note, here, that the prediction model 22 may be a prediction model which has been generated by a machine learning process in which at least any one of the following (1) and (2) is used as training data.

(1) A combination of near-infrared measurement data and physical property information, the near-infrared measurement data containing near-infrared absorption spectra of a plurality of produced resin powders which have been previously produced and have each known (measured) physical property, the physical property information being on end products each of which is associated with the near-infrared measurement data.

(2) A combination of near-infrared measurement data and physical property information, the near-infrared measurement data containing near-infrared absorption spectra of a plurality of produced intermediate products which have been produced in a process for producing a corresponding one of the plurality of produced resin powders and have each known (measured) physical property, the physical property information being on the plurality of produced intermediate products each of which is associated with the near-infrared measurement data.

In an example, the prediction model 22 which has been trained may be introduced in the predicting apparatus 100 in advance. Alternatively, the predicting apparatus 100 may further has a function of carrying out the machine learning process in which at least any one of the above (1) and (2) is used as training data.

With use of the prediction model 22 generated by such machine learning, the predicting apparatus 100 is capable of accurately predicting, from a near-infrared absorption spectrum, the physical property of the resin powder with respect to which the near-infrared absorption spectrum has been measured. A method for generating the prediction model 22 will be described later.

As illustrated in Fig. 2, the predicting apparatus 100 includes a control section 10 which integrally controls each section of the predicting apparatus 100, a storage section 20 in which various pieces of data used by the control section 10 are stored, and a communication section 50 which is for outputting a prediction result to the external apparatus 4. The control section 10 corresponds to the CPU 1 illustrated in Fig. 1, and the storage section 20 corresponds to the memory 2 illustrated in Fig. 1.

The communication section 50 is for carrying out data communications with the external apparatus 4. Communications between the predicting apparatus 100 and the external apparatus 4 may be carried out via a network such as near field communication, wired connection, and the Internet. Alternatively, the predicting apparatus 100 and the external apparatus 4 may be directly connected by a connector such as a USB terminal.

The control section 10 includes a measurement data obtaining section 11 and a predicting section 13.

The measurement data obtaining section 11 obtains the near-infrared absorption spectrum from the near-infrared spectrophotometer 3. The measurement data obtaining section 11 may store, in the storage section 20, the obtained near-infrared absorption spectrum as near-infrared measurement data (not illustrated). The measurement data obtaining section 11 may also read out, from the near-infrared measurement data, a near-infrared absorption spectrum which has been previously stored, and use the near-infrared absorption spectrum for subsequent prediction.

The predicting section 13 inputs the near-infrared absorption spectrum into the prediction model 22 as described later, and outputs prediction information concerning the physical property of the resin powder.

The predicting section 13 may generate one or more pieces of processed data on the basis of the near-infrared absorption spectrum. Note, here, that the one or more pieces of processed data are different from raw data on the near-infrared absorption spectrum, and are data obtained by carrying out one or more given preprocesses with respect to the near-infrared absorption spectrum. The predicting section 13 may carry out the one or more preprocesses, which are specified in the prediction model 22, with respect to the near-infrared absorption spectrum obtained by the measurement data obtaining section 11 (preprocessing step). The one or more preprocesses include at least one of the following processes.

· Outlier removal process

An outlier removal process is a process for, in a case where near-infrared absorption spectra measured at a plurality of portions of a resin powder are compared with each other, detecting a near-infrared absorption spectrum which significantly differs from the other near-infrared absorption spectra and removing the near-infrared absorption spectrum. Note that the expression "measured at a plurality of portions of a resin powder" is equivalent to irradiating, with measurement light, a plurality of different regions of a measurement-target sample which is constituted by a resin powder and which has a given area and carrying out measurement. Specific examples of a method for detecting an outlier include the one-class support vector machine process (One-Class SVM process), detection with use of the Mahalanobis distance, the local outlier factor (LOF), the Tukey method, and the nearest neighbor method.

· Averaging process

An averaging process is a process of calculating a single piece of average spectral data from a plurality of near-infrared absorption spectra measured at a plurality of portions of a resin powder.

· Wavelength selection process

A wavelength selection process is a process of selecting a wavelength range of spectral data to be inputted into the prediction model 22 (described later). In this wavelength range process, for example, a wavelength range in which a characteristic absorption pattern is shown may be selected for each resin powder with respect to which a near-infrared absorption spectrum has been measured.

· Differential process

A differential process is a process of generating differential data which is obtained by differentiation of spectral data with respect to a wavelength. The differential data may include data which is obtained by first-order differentiation of spectral data with respect to a wavelength and data which is obtained by second-order differentiation of the spectral data with respect to the wavelength.

· Baseline correction process

A baseline correction process is a process of aligning baselines of a plurality of near-infrared absorption spectra measured at a plurality of portions of a resin powder.

The above-listed preprocesses are merely examples, and the one or more preprocesses carried out by the predicting section 13 are not limited to these preprocesses. For example, the predicting section 13 may carry out any of the following processes with respect to the near-infrared absorption spectrum.

    · Smoothing process (weighted moving average process, smoothing spline process, and the like)
    · Difference spectrum process

· Standard normal variate (SNV) process
· Multiple scattering correction (MSC) process
Dimensionality reduction by principal component analysis (PCA)

Any of the other processes such as classification and clustering may be carried out.

A predicting method carried out by the predicting section 13 may include, for example, an averaging step of calculating average spectral data by: obtaining a plurality of pieces of near-infrared measurement data which indicate a plurality of near-infrared absorption spectra of the resin powder, at a plurality of portions of the resin powder; and carrying out an averaging process with respect to the obtained plurality of near-infrared absorption spectra. In the predicting step, the average spectral data may be inputted, as processed data, into the prediction model 22. The averaging step may be a process specified by the prediction model 22.

The predicting method carried out by the predicting section 13 may further include, for example, a wavelength range selecting step of selecting a wavelength range of the average spectral data to be inputted into the prediction model 22. In the predicting step, the average spectral data in the wavelength range may be inputted, as processed data, into the prediction model 22. The wavelength range selecting step may be a process specified by the prediction model 22.

The predicting method carried out by the predicting section 13 may further include, for example, a differential data generating step of generating differential data which is obtained by differentiation of the average spectral data in the above-described wavelength range with respect to a wavelength. In the predicting step, the differential data may be inputted, as processed data, into the prediction model 22. The differential data generating step may be a process specified by the prediction model 22.

(Process carried out by predicting apparatus 100)

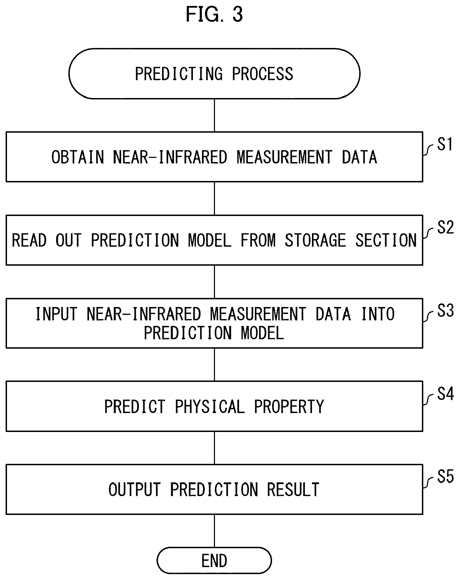

The following description will discuss a process carried out by the predicting apparatus 100, with reference to Fig. 3. Fig. 3 is a flowchart illustrating a flow of the process carried out by the predicting apparatus 100.

Firstly, the measurement data obtaining section 11 obtains near-infrared measurement data which is a near-infrared absorption spectrum measured by the near-infrared spectrophotometer 3 (step S1: near-infrared measurement data obtaining step).

Next, the predicting section 13 reads out the prediction model 22 from the storage section 20 (step S2).

The predicting section 13 inputs, into the prediction model 22, the near-infrared measurement data obtained in step S1 (step S3). In this case, the predicting section 13 may carry out, on the basis of the prediction model 22, one or more preprocesses with respect to the obtained near-infrared absorption spectrum. The predicting section 13 may carry out one of the above-listed preprocesses or may alternatively carry out two or more of the above-listed preprocesses. The one or more preprocesses carried out by the predicting section 13 will be described later with reference to a specific example.

Next, on the basis of the prediction model 22, the predicting section 13 predicts a physical property of a prediction target from the near-infrared measurement data which has been preprocessed or the near-infrared measurement data which is unprocessed (step S4: predicting step).

The communication section 50 outputs, to the external apparatus 4, prediction information which indicates a prediction result outputted from the predicting section 13 (step S4).

<Example of preprocesses>

As an example, specific preprocesses carried out by the predicting section 13 in a case where gel D50 is predicted from a plurality of near-infrared absorption spectra of a hydrogel, which is an intermediate product in a process for producing a resin powder, will be described here.

The predicting section 13 carries out an outlier detection process (e.g., One Class SVM or the like) with respect to the plurality of near-infrared absorption spectra, which have been obtained by the measurement data obtaining section 11, so as to remove a near-infrared absorption spectrum which significantly differs from the other near-infrared absorption spectra.

Next, the predicting section 13 carries out an averaging process with respect to a plurality of remaining near-infrared absorption spectra so as to generate a single piece of average spectral data.

Note that the one or more preprocesses carried out by the predicting section 13 may vary depending on in which stage in a process for producing the resin powder the near-infrared absorption spectrum has been obtained and what physical property is to be predicted. That is, the predicting section 13 may carry out a wavelength selection process of selecting a wavelength range of spectral data. Alternatively, the predicting section 13 may carry out a differential process of generating differential data which is obtained by differentiation of spectral data with respect to a wavelength. Note also that these processes may be carried out in combination.

In this manner, it is possible to increase prediction accuracy of the predicting apparatus 100 by carrying out an appropriate preprocess(es) depending on in which stage in a process for producing a resin powder a near-infrared absorption spectrum has been obtained and what physical property is to be predicted.

(Configuration of predicting apparatus 100)

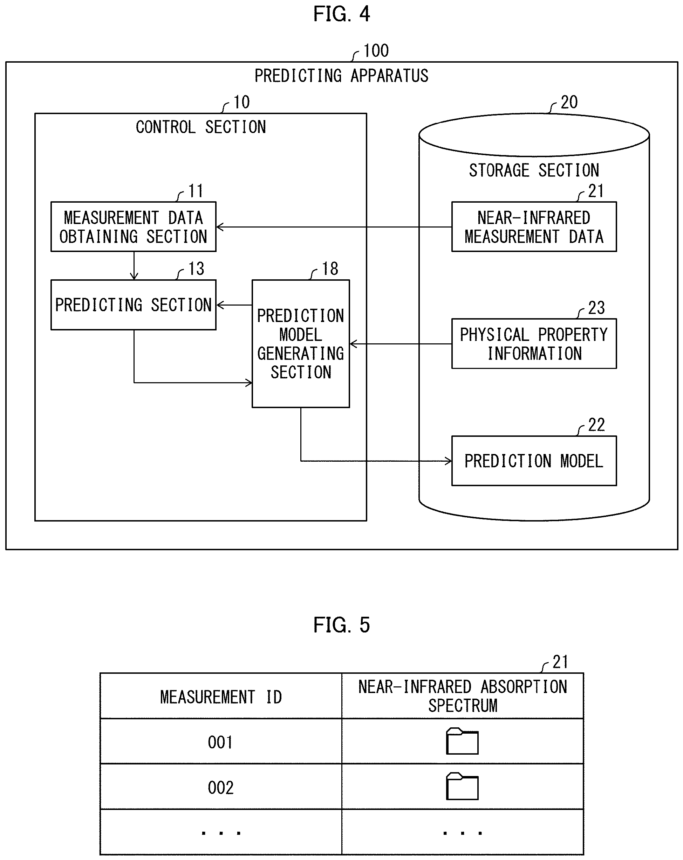

Next, a configuration of the predicting apparatus 100 which carries out machine learning for generating the prediction model 22 will be described with reference to Fig. 4. Fig. 4 is a functional block diagram illustrating an example of a configuration of a main part of the predicting apparatus 100 which generates the prediction model 22. For convenience, members having the same functions as members described with reference to Fig. 1 are given the same reference signs and description thereof will not be repeated. Note that the predicting apparatus 100 may generate the prediction model 22 by carrying out any known supervised machine learning.

The control section 10 includes the measurement data obtaining section 11, the predicting section 13, and a prediction model generating section 18.

The measurement data obtaining section 11 obtains a plurality of near-infrared absorption spectra (also referred to as "near-infrared absorption spectrum group") which are contained in near-infrared measurement data 21 and which have been specified by the prediction model generating section 18, and outputs the near-infrared absorption spectrum group to the predicting section 13.

The predicting section 13 reads out, from the prediction model generating section 18, a prediction model candidate (described later) generated by the prediction model generating section 18. Furthermore, the predicting section 13 inputs, into the prediction model candidate, the near-infrared absorption spectrum group which is contained in the near-infrared measurement data 21 and which has been specified by the prediction model generating section 18, and outputs, to the prediction model generating section 18, a prediction result of predicting a physical property group corresponding to the inputted near-infrared absorption spectrum group.

The prediction model generating section 18 generates the prediction model candidate, with respect to which training and validation in machine learning are carried out. Note that the prediction model candidate is a prediction model with respect to which prior machine learning has not been completed. In a case where given machine learning is completed and prediction accuracy satisfies a criterion, the prediction model candidate is stored in the storage section 20 as the prediction model 22. Further, the prediction model generating section 18 specifies, from the near-infrared measurement data 21 and physical property information 23 which are stored in the storage section 20, data groups to be subjected to the machine learning.

The prediction model generating section 18 may calculate a model evaluation index by comparing the following (1) and (2).

(1) The prediction result which is obtained by predicting the physical property group with use of the prediction model candidate and which has been outputted from the predicting section 13.
(2) The physical property group which is associated with the near-infrared absorption spectrum group that has been inputted into the prediction model candidate and which is included in the physical property information 23 that has been read out from the storage section 20.

Note, here, that the model evaluation index is an index for evaluating an error between the prediction result in (1) and the physical property group which is included in the physical property information 23 in (2), for example. The model evaluation index may be any index which makes it possible to evaluate accuracy of the prediction result. The model evaluation index may be a mean square error or may be alternatively a coefficient of determination ($R^2$).

The prediction model generating section 18 determines, on the basis of the model evaluation index, whether or not the prediction model candidate satisfies a given evaluation criterion. The given evaluation criterion is a criterion which is predetermined as desired so that prediction accuracy of the prediction model candidate is evaluated.

In a case where the prediction model candidate satisfies the given evaluation criterion, the prediction model generating section 18 stores the prediction model candidate, as an optimal prediction model, in the prediction model 22. In a case where the generated prediction model candidate does not satisfy the given evaluation criterion, the prediction model generating section 18 carries out update of the prediction model candidate.

The "update of the prediction model candidate" may include updating the prediction model candidate by updating a weight, a hyperparameter, and/or the like of the prediction model candidate so that the error between the prediction result and the physical property group which is included in the physical property information 23 is minimized, and also may include generating a new prediction model candidate. A backpropagation method or the like may be employed to update the prediction model candidate.

The physical property information 23 includes physical property information on end products each of which is associated with the near-infrared measurement data containing near-infrared absorption spectra of a plurality of produced resin powders which have been previously produced and have each known (measured) physical property. Further, the physical property information 23 includes physical property information on a plurality of produced intermediate products each of which is associated with the near-infrared measurement data 21 containing near-infrared absorption spectra of the plurality of produced intermediate products which have been produced in a process for producing a corresponding one of the plurality of produced resin powders and have each known (measured) physical property. The physical property information can be information concerning the physical property of the water absorbent resin powders (described later).

The physical property information 23 may include, as the physical property, actually measured values of the resin powders or the intermediate products which have become measurement targets. Each physical property may be given a measurement ID. The physical property information 23 may include the physical property group which is used in the machine learning for generating the prediction model 22 from the prediction model candidate.

The near-infrared measurement data 21 includes data files of the near-infrared absorption spectra of the resin powders or the intermediate products which have become measurement targets. These data files can be each, for example, a csv file or a text file. The data files of the near-infrared absorption spectra may be each given a measurement ID. The near-infrared measurement data 21 may include the near-infrared absorption spectrum group which is used in the machine learning for generating the prediction model 22 from the prediction model candidate.

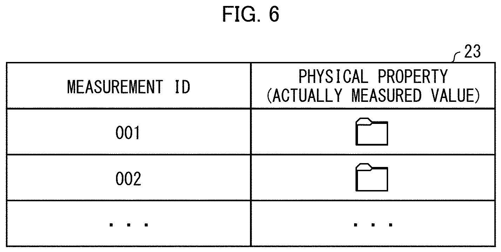

Here, correspondence between the near-infrared absorption spectra contained in the near-infrared measurement data 21 and the physical property included in the physical property information 23 will be described with reference to Figs. 5 and 6.

Fig. 5 is a figure illustrating a data structure of the near-infrared measurement data 21. Fig. 6 is a figure illustrating a data structure of the physical property information 23. In Fig. 5, the near-infrared measurement data 21 has the data files of the near-infrared absorption spectra, and the data files of the near-infrared absorption spectra are each given a measurement ID.

In Fig. 6, the physical property information 23 has data files of the physical property (actually measured values), and each physical property is given a measurement ID. As illustrated in Fig. 6, this measurement ID may be the same as the above-described measurement ID which is given to a corresponding one of the data files of the near-infrared absorption spectra. A near-infrared absorption spectrum and physical property information which are given the same ID may be results of carrying out measurement with respect to the same product. For example, the near-infrared absorption spectrum which is given a measurement ID "001" in Fig. 5 and the physical property which is given a measurement ID "001" in Fig. 6 may be data obtained by carrying out measurement with respect to the same resin powder or intermediate product.

The predicting section 13 uses the prediction model candidate specified by the prediction model generating section 18. The predicting section 13 may obtain the measurement IDs of the near-infrared absorption spectra specified by the prediction model generating section 18, and compare the prediction result with the physical property group which corresponds to the near-infrared absorption spectrum group that has been read out from the storage section 20 and which have the same measurement IDs as the above measurement IDs, as information concerning the same products. The prediction result outputted from the predicting section 13 may be given the same measurement IDs as the measurement IDs of the near-infrared absorption spectra which have been inputted into the prediction model candidate. The prediction model generating section 18 may compare the prediction result outputted from the predicting section 13 with the physical property group which corresponds to the near-infrared absorption spectrum group that has been read out from the storage section 20 and which have the same measurement IDs as the prediction result, as information concerning the same products.

Note that the measurement ID given to each physical property may differ from the measurement ID given to a corresponding one of the near-infrared absorption spectra. In a case where the measurement ID given to each physical property differs from the measurement ID given to a corresponding one of the near-infrared absorption spectra, these measurement IDs only need to be associated with each other.

The prediction model 22 may be a prediction model which has been generated with use of any one of the following types of machine learning: linear regression; and non-linear regression. As the machine learning for generating the prediction model 22, examples of the linear regression include partial least squares (PLS) regression, principal component regression (PCR), simple regression, multiple regression, ridge regression, lasso regression, and Bayesian linear regression. Examples of the non-linear regression include a (convolutional) neural network, support vector regression, a k-nearest neighbor method, and a regression tree. Ensemble learning in which any of the above-listed methods are combined may be used. The prediction model 22 may be a model for carrying out numerical value prediction in which various physical property values are predicted, or may be a model for carrying out determination prediction in which it is determined whether the physical property values are acceptable or not.

In an embodiment of the present disclosure, machine learning of PLS and PCR are preferably used to generate the prediction model 22.

The prediction model 22 may specify a necessary preprocess(es) to be carried out with respect to the near-infrared absorption spectra.

(Process of generating prediction model 22)

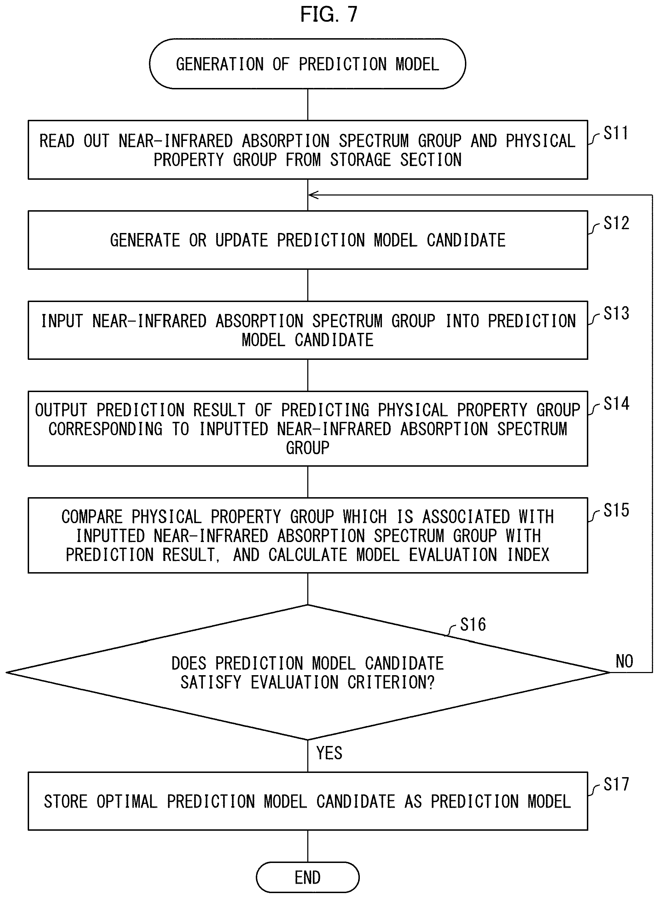

Next, a process carried out by the predicting apparatus 100 will be described with reference to Fig. 7. Fig. 7 is a flowchart illustrating a flow of the process carried out by the predicting apparatus 100 which carries out the machine learning. Note that, here, an example will be given in which the predicting apparatus 100 generates the prediction model 22 with use of, as training data, a combination of the near-infrared measurement data 21 and the physical property information 23 corresponding to the near-infrared measurement data 21.

Firstly, the measurement data obtaining section 11 reads out, from the storage section 20, a near-infrared absorption spectrum group which is contained in the near-infrared measurement data 21 and which is specified by the prediction model generating section 18 for use in a prediction model candidate. The measurement data obtaining section 11 further reads out a physical property group which is associated with the near-infrared absorption spectrum group and included in the physical property information 23 (step S11).

Next, the prediction model generating section 18 generates a prediction model candidate and outputs the prediction model candidate to the predicting section 13 (step S12).

The predicting section 13 inputs, into the prediction model candidate, the near-infrared absorption spectrum group which the measurement data obtaining section 11 obtained (step S13).

The predicting section 13 outputs a prediction result of predicting a physical property group corresponding to the near-infrared absorption spectrum group which has been inputted into the prediction model candidate (step S14).

The prediction model generating section 18 compares the physical property group which is associated with the inputted near-infrared absorption spectrum group with the prediction result which has been outputted from the predicting section 13, and calculates a model evaluation index (step S15).

The prediction model generating section 18 determines, in reference to the model evaluation index, whether the prediction model candidate satisfies a given evaluation criterion (step S16). In a case where the prediction model candidate satisfies the given evaluation criterion (YES in the step S16), the prediction model generating section 18 stores the prediction model candidate, as an optimal prediction model candidate, in the prediction model 22 (step S19).

In a case where the prediction model candidate does not satisfy the given evaluation criterion (NO in the step S16), the prediction model generating section 18 updates the prediction model candidate (step S12). Note that the prediction model generating section 18 may update, in the step S12, a weight, a hyperparameter, and/or the like of the prediction model candidate which does not satisfy the evaluation criterion, or may generate a new prediction model candidate.

The process in the steps S12 to S16 is repeated until the step S16 reaches YES.

In Fig. 7, the process in which the prediction model generating section 18 generates a single prediction model candidate and generates the prediction model 22 by the machine learning from the single prediction model candidate was described as an example. However, the prediction model generating section 18 is not limited to such a configuration. For example, the prediction model generating section 18 may generate a plurality of prediction model candidates. In this case, the prediction model generating section 18 may be configured to, after carrying out the machine learning illustrated in Fig. 7 with respect to each of the plurality of prediction model candidates, store a prediction model candidate with the highest prediction accuracy in the prediction model 22 as an optimal prediction model candidate. Further, the prediction model generating section 18 may carry out the machine learning so as to generate a prediction model including a preprocess(es).

(Measurement method for obtaining near-infrared absorption spectrum)

A method for measuring a near-infrared absorption spectrum according to an embodiment of the present disclosure is a method for measuring a near-infrared absorption spectrum of a resin powder for use in the above-described prediction method carried out by the predicting apparatus 100. The measurement method includes: a step of irradiating a resin powder with near-infrared radiation; and a step of calculating a near-infrared absorption spectrum of the resin powder from a measurement value obtained by measuring at least one of light reflected by the resin powder and light transmitted by the resin powder. The resin powder is any one of a water absorbent resin powder and an intermediate product which is produced in a process for producing the water absorbent resin powder. The near-infrared absorption spectrum will be described below.

(Near-infrared absorption spectrum)

Here, a near-infrared absorption spectrum which is used by the predicting apparatus 100 to predict a physical property of a resin powder will be described.

<Measurement apparatus>

The near-infrared absorption spectrum is measured by a near-infrared spectrophotometry in which a sample is irradiated with near-infrared radiation in a specific wavelength range and transmitted light or reflected light is detected. The near-infrared absorption spectrum can be measured, for example, with use of a near-infrared spectrophotometer. Examples of the near-infrared spectrophotometer include, but are not particularly limited to, FT-NIR NIRFlex (registered trademark) N-500 series and NIRMaster series (manufactured by BUCHI), IRMA51 series and IRMD51 series (manufactured by Chino Corporation), IR Tracer100 NIR system (manufactured by Shimadzu Corporation), Spectrum3 NIR (manufactured by PerkinElmer), and MATRIX series FT-NIR spectrometers (manufactured by BRUKER). Commercial software can be used to analyze obtained spectral data. Note that near-infrared spectrophotometers may be shown in different names, such as a near-infrared multi-component analyzer and a near-infrared analyzer, depending on manufacturers.

<Wavelength of near-infrared radiation>

The near-infrared radiation is light that has a wavelength falling within a wavelength range of 750 nm to 2500 nm. The near-infrared radiation spectrum is measured by irradiation with light which contains near-infrared radiation that has a wavelength falling within the above wavelength range. Such irradiation light may have all wavelengths falling within the near-infrared wavelength range, or may have one or more selected specific wavelengths. In measurement of the near-infrared absorption spectrum in an embodiment of the present disclosure, a water absorbent resin, which is a measurement target, is irradiated with the above irradiation light, and transmitted, absorbed, refracted, reflected, and/or diffused light is measured. Therefore, it is possible to collect not only chemical information but also physical information. In other words, the near-infrared absorption spectrum is affected by the temperature of such a measurement target sample, an atmosphere (e.g., whether or not steam is present, whether or not replacement by nitrogen is present, air pressure, and the like) inside a measurement light path, surface roughness, the thickness of the sample, a state of filling of the sample, and a time period before the measurement. Thus, from the viewpoint of prediction accuracy, in obtaining the near-infrared absorption spectrum, it is preferable to measure the near-infrared absorption spectrum under the condition that the above physical conditions are as uniform as possible. As necessary, a physical condition (e.g., the temperature of the sample) may be measured separately, and a corresponding near-infrared absorption spectrum may be corrected on the basis of a measured value.

In an embodiment of the present disclosure, the near-infrared absorption spectrum is measured at at least any one of the following points in time: before a polymerization step; between the polymerization step and a drying step; and after the drying step. The above-described prediction information outputted in the predicting step may be used to control any one or more production apparatuses which are used in a process for producing the resin powder.

In an embodiment of the present disclosure, the merits of using the near-infrared absorption spectrum include the following: (1) an analysis result is obtained quickly; (2) an analysis is carried out in a noncontact and nondestructive manner; (3) quantitative analyses of multiple components can be carried out at the same time; (4) a physical quantity (e.g., particle size or the like) can be measured; and (5) an operation is easy.

(Physical properties of water absorbent resin powder)

In the predicting method according to an embodiment of the present disclosure, prediction information concerning a physical property of at least any one of a water absorbent resin powder and an intermediate product which is produced in a process for producing the water absorbent resin powder is outputted.

The physical property which can be predicted by the predicting apparatus 100 may include at least any one of the following (1) to (16).

    (1) Gel D50
    (2) CRC
    (3) AAP
    (4) SFC
    (5) T20, U20, K20
    (6) Vortex
    (7) D50
    (8) Moisture content of hydrogel
    (9) Solid fraction
    (10) Residual Monomers
    (11) FSR

(12) FSC
(13) Flow Rate
(14) Density
(15) Ext
(16) Gel Ext

The physical property which can be predicted by the predicting apparatus 100 is preferably at least one of (1) gel D50, (2) CRC, (3) AAP, (4) SFC, (6) Vortex, (7) D50, (8) the moisture content of a hydrogel, and (9) a solid fraction.

<Water absorbent resin>

The term "water absorbent resin" in an embodiment of the present disclosure means a crosslinked polymer having a water-swelling property and water-insolubility, and the water absorbent resin is generally particulate. The term "water-swelling property" means an absorption capacity without load (CRC), as defined in NWSP 241.0.R2 (15), of 5 g/g or more. The term "water-insolubility" means a soluble content (Ext), as defined in NWSP 270.0.R2 (15), of 50 mass% or less.
The water absorbent resin can be designed as appropriate according to the purpose of use thereof, and is not limited to any particular design. The water absorbent resin is preferably a hydrophilic crosslinked polymer that has been obtained by polymerizing and crosslinking unsaturated monomers each of which has a carboxyl group. Moreover, the water absorbent resin is not limited to a form in which the water absorbent resin is wholly (that is, 100 weight%) a polymer, and can be a water absorbent resin that is surface-crosslinked or a water absorbent resin composition that contains an additive and/or the like, within a range in which the above-described performance is maintained.
As an example, the "water absorbent resin" is a "poly(meth)acrylic acid (salt)", and may contain, as a main component, a (meth)acrylic acid and/or a salt thereof as a repeating unit.
The "NWSP" represents the "Non-Woven Standard Procedures-Edition 2015", which refers to evaluation methods that are for nonwoven fabrics and products thereof and that each have been standardized and jointly issued in the United States and Europe by the European Disposales And Nonwovens Associations (EDANA) and the Association of the Nonwoven Fabrics Industry (INDA). The NWSP shows a standard method for measuring a water absorbent resin. In an embodiment of the present disclosure, the physical property of the water absorbent resin is measured in conformity with the "NWSP", unless otherwise specified.

<Gel D50>

The gel D50 is a mass average particle diameter in terms of a solid content of a hydrogel which is an intermediate product. The gel D50 is measured in conformity with the method disclosed in WO2016/204302. The gel D50 in an embodiment of the present disclosure indicates a value corresponding SolidD50 described in WO2016/204302.
In an embodiment of the present disclosure, the gel D50 can be measured after the polymerization step and before the drying step. In a case where the water absorbent resin is produced by aqueous solution polymerization, the gel D50 is measured after a gel-crushing step (described later) or before the drying step.

<CRC> (NWSP 241.0.R2 (15))

The term "CRC" is an acronym for "centrifuge retention capacity", and means a fluid retention capacity without pressure (hereinafter, referred to also as "fluid retention capacity") of a water absorbent resin.
Specifically, the CRC refers to a fluid retention capacity (unit: g/g) measured after 0.2 g of a water absorbent resin contained in a nonwoven fabric bag is immersed in a large excess of a 0.9 weight% aqueous sodium chloride solution for 30 minutes so as to be allowed to freely swell and then the water absorbent resin is drained in a centrifuge (250 G).

<AAP> (NWSP 242.0.R2 (15))

The term "AAP" is an acronym for "absorption against pressure", and means a fluid retention capacity under pressure of a water absorbent resin.
Specifically, the AAP refers to a fluid retention capacity (unit: g/g) measured after 0.9 g of a water absorbent resin has been swollen in a large excess of a 0.9 weight% aqueous sodium chloride solution for 1 hour under a load of 2.06 kPa (21 g/cm2, 0.3 psi). Note that in some cases the measurement may be carried out under a load of 4.83 kPa (49 g/cm$^2$, 0.7 psi).

<SFC>

The term "SFC" is an acronym for "saline flow conductivity", and refers to liquid permeability (unit: $\times$ $10^{-7} \cdot cm^3 \cdot s \cdot g^{-1}$) of a 0.69 weight% aqueous sodium chloride solution in a water absorbent resin under a load of 2.07 kPa. The "SFC" is measured in conformity with the SFC test method disclosed in U.S. Patent No. 5669894.

<T20>

The term "T20" refers to a water absorption time, and refers to a time period (unit: second) required for 1 g of a resin powder to absorb 20 g of a 0.9 weight% aqueous sodium chloride solution. The T20 is measured in conformity with the measurement method disclosed in the U.S. Patent Application Publication No. US 2012/0318046.

<U20>

The term "U20" refers to an absorption (unit: g/g) in 20 minutes. The U20 is measured in conformity with the measurement method disclosed in the U.S. Patent Application Publication No. US 2012/0318046.

<K20>

The term "K20" refers to effective permeability (unit: $m^2$) in 20 minutes. The K20 is measured in conformity with the measurement method disclosed in the U.S. Patent Publication, US2012/0318046.

<Vortex>

The term "Vortex" (water absorption time) is measured in accordance with the following procedure. Firstly, 0.02 parts by mass of food blue No. 1 (brilliant blue), which is a food additive, is added to 1000 parts by mass of preadjusted physiological saline (0.9 mass% aqueous sodium chloride solution). Then, the temperature of the physiological saline is adjusted to 30°C.
Next, 50 ml of the physiological saline was measured and put in a 100-ml beaker. While the physiological saline is being stirred at 600 rpm with use of a stirrer tip having a length of 40 mm and a diameter of 8 mm, 2.0 g of a water absorbent resin is introduced into the physiological saline. A point in time of the introduction of the water absorbent resin is regarded as a starting point, and a time period required for the water absorbent resin to absorb the physiological saline and cover the stirrer tip is measured as Vortex (water absorption time) (unit: second).

<D50>

In an embodiment of the present disclosure, the term "D50" refers to a mass average particle diameter of a resin powder produced in the drying step (described later). The mass average particle diameter (D50) is measured by a method similar to the method described in the section "(3) Mass-Average Particle Diameter (D50) and Logarithmic Standard Deviation of Particle Diameter Distribution" in US Patent No. 7638570.

<Moisture content and solid fraction of hydrogel> (NWSP 230.0.R2)

The moisture content and the solid fraction of a hydrogel refer to the moisture content and the resin solid fraction, respectively, of a hydrogel which has not been dried. The moisture content and the solid fraction of the hydrogel can be measured after the polymerization step and before the drying step. That is, the moisture content and the solid fraction of the hydrogel may be the moisture content and the solid fraction, respectively, of the hydrogel which has not been crushed, or may be the moisture content and the solid fraction, respectively, of the hydrogel which has been crushed so as to be particulate.
The moisture content of the hydrogel is measured in conformity with the NWSP. Note that, in the measurement, the mass of a sample is changed to 2.0 g, a drying temperature is changed to 180°C, and a drying time is changed to 24 hours. Specifically, after 2.0 g of the hydrogel is introduced into an aluminum cup having a 50-mm diameter bottom surface, the total mass W1 (g) of the sample (the hydrogel and the aluminum cup) is accurately weighed. Next, the sample is allowed to stand still in an oven in which an ambient temperature is set to 180°C. After the elapse of 24 hours, the sample is taken out from the oven, and the total mass W2 (g) is accurately weighed. The mass of the hydrogel subjected to the measurement is regarded as M (g), and the moisture content (100-$\alpha$) (mass%) of the hydrogel is calculated in accordance with (Equation 1) below. Note that $\alpha$ is the solid fraction (mass%) of the hydrogel.

$$(100-\alpha)\ (mass\%)=\{(W1-W2)/M\}\times 100\cdots(Equation\ 1)$$

<Gel CRC>

The term "gel CRC" refers to the CRC of a hydrogel which has not been dried. The gel CRC can be measured after the polymerization step and before the drying step. That is, the gel CRC may be the CRC of the hydrogel which has not been crushed, or may be the CRC of the hydrogel which has been crushed so as to be particulate.

Specifically, the "gel CRC" refers to a fluid retention capacity (unit: g/g) measured after 0.6 g of a hydrogel contained in a nonwoven fabric bag is immersed in a large excess of a 0.9 weight% aqueous sodium chloride solution for 24 hours so that the water absorbent resin is allowed to freely swell and then the water absorbent resin is drained in a centrifuge (250 G).

<Ext> (NWSP 270.0.R2 (15))

The term "Ext" is an abbreviation for "extractables", and means a water-soluble content (water-soluble component amount). Specifically, the Ext refers to the amount (unit: weight%) of a dissolved polymer measured after 1.0 g of a water absorbent resin is added to 200 mL of a 0.9 weight% aqueous sodium chloride solution and then a resulting solution is stirred for 16 hours. The amount of the dissolved polymer is measured by pH titration.

<Gel Ext>

The term "gel Ext" is the Ext of a hydrogel which has not been dried. The gel Ext can be measured after the polymerization step and before the drying step. That is, the gel Ext may be the Ext of the hydrogel which has not been crushed, or may be the Ext of the hydrogel which has been crushed so as to be particulate.

Specifically, on the basis of the above-described method for measuring the "Ext", the amount of a sample is changed to 2.0 g and then the gel Ext is measured. The gel Ext is calculated as mass% of a water-soluble content per solid content.

<Residual Monomers> (NWSP 210.0.R2 (19))

The term "Residual Monomers" refers to the amount of monomers remaining in the water absorbent resin. The Residual Monomers are measured in conformity with NWSP 210.0.R2 (19).

<FSR>

The term "FSR" refers to a water absorption speed (unit: g/g/s). The FSR is measured in conformity with the measurement method disclosed in the International Publication No. WO 2009/016055.

<FSC> (NWSP 240.0.R2 (15))

The term "FSC" is an acronym for "free swell capacity", and means a fluid retention capacity without load after suspension of a water absorbent resin. The FSC is measured in conformity with NWSP 240.0.R2 (15).

<Flow Rate> (NWSP251.0.R2 (15))

The term "Flow Rate" means a flow speed of a water absorbent resin. The Frow Rate is measured in conformity with NWSP 251.0.R2 (15).

<Density>

The term "Density" means the bulk density of a water absorbent resin. The Density is measured in conformity with NWSP 251.0.R2 (15).

The above-described physical properties may be each measured at any timing in the process for producing the resin powder.

(Method for producing resin powder)

The above-described physical properties of a resin powder are each measured in a process for producing the water absorbent resin powder. The following description will discuss a method for producing a resin powder.

In an embodiment of the present disclosure, a known method or a combination of known methods can be used as the method for producing a water absorbent resin powder. As an example, the method for producing a water absorbent resin powder only needs to include the polymerization step and the drying step. As a preferable example, the method may include the gel-crushing step, a post-crosslinking step, and a sizing step. Each step will be described below.

<Polymerization step>

The polymerization step is, as an example, a step of polymerizing an aqueous monomer solution which contains a monomer and at least one polymerizable internal crosslinking agent to obtain a crosslinked hydrogel polymer (hereinafter referred to as "hydrogel"). The monomer contains acrylic acid (salt) as a main component.

[Polymerization initiator]

A polymerization initiator used in an embodiment of the present disclosure is selected as appropriate in accordance with a form of polymerization or the like, and is therefore not limited to any particular one. Examples of the polymerization initiator include pyrolysis-type polymerization initiators, photolytic-type polymerization initiators, and redox-type polymerization initiators containing a reducing agent for facilitating decomposition of any of those polymerization initiators. Specifically, one of or two or more of the polymerization initiators disclosed in U.S. Patent No. 7265190 are used. From the viewpoint of the handleability of the polymerization initiator and the physical properties of a particulate water-absorbing agent or a water absorbent resin, the polymerization initiator is preferably a peroxide or an azo compound, more preferably a peroxide, and even more preferably a persulfate.

Note that a polymerization reaction may be carried out by, instead of using the polymerization initiator, irradiating the monomer with an active energy ray such as a radial ray, an electron ray, or an ultraviolet ray. Alternatively, any of these active energy rays may be used in combination with the polymerization initiator.

[Form of polymerization]

The polymerization applied to an embodiment of the present disclosure is not limited to any particular form. From the viewpoint of the water absorbent property of a hydrogel, ease of control of the polymerization, and the like, preferable examples of the polymerization include spray droplet polymerization, aqueous solution polymerization, and reversed phase suspension polymerization. More preferable examples of the polymerization include aqueous solution polymerization and reverse phase suspension polymerization. Even more preferable examples of the polymerization include aqueous solution polymerization. Among these, continuous aqueous solution polymerization is particularly preferable. The continuous aqueous solution polymerization can be any one of continuous belt polymerization and continuous kneader polymerization.

<Gel-crushing step>

The gel-crushing step is a step of gel-crushing the hydrogel, obtained in the polymerization step, to obtain a particulate hydrogel. In a case where a water absorbent resin is produced by spray droplet polymerization or reversed phase suspension polymerization, a particulate hydrogel can be obtained. In this case, it is not necessary to carry out the gel-crushing step. Further, the gel-crushing step may be carried out simultaneously with the polymerization step, as in continuous kneader polymerization. In particular, from the viewpoint of obtaining a SAP having a high water absorption speed, it is preferable to, in the gel-crushing step, refine the hydrogel to produce a particulate hydrogel having gel D50 falling within a desired range.

[Gel-crusher]

Examples of a gel-crushing apparatus used during or after the polymerization in this step include, but are not particularly limited to, gel-crushers provided with a plurality of rotary stirring blades (such as batch-type and continuous-type twin-arm kneaders), single-screw extruders, twin-screw extruders, meat choppers, screw-type extruders, and multi-screw kneaders provided with a crushing means.

Among these, a screw-type extruder in which a porous plate is placed at one end of a casing is preferable. Specific examples of such a screw-type extruder are disclosed in Japanese Patent Application Publication Tokukai No.

2000-63527 and WO2011/126079.

[Gel-crushing region]

In an embodiment of the present disclosure, the above gel-crushing is carried out during and/or after the polymerization step, and is more preferably carried out with respect to the hydrogel polymer after the polymerization step. In a case where the gel-crushing is carried out during the polymerization as in kneader polymerization and the like, the aqueous monomer solution continuously changes into a hydrogel polymer as polymerization progresses. Thus, it is only necessary to gel-crush the hydrogel polymer at/after a point in time at which the maximum polymerization temperature is reached or gel-crush the hydrogel polymer in which a rate of polymerization of the monomer reaches 90 mol% or more. Note, here, that the maximum polymerization temperature is also referred to as a polymerization peak temperature. Note also that the rate of polymerization of the monomer is also referred to as a rate of conversion. The rate of polymerization of the monomer is calculated from the amount of a residual monomer and the amount of a polymer which is calculated by pH titration of the hydrogel polymer.

In a case where the polymerization step is carried out by belt polymerization, the hydrogel polymer during and/or after the polymerization step, preferably after the polymerization step, can be chopped or broken to a size of approximately several tens of centimeters prior to the gel-crushing. This operation makes it easily to feed the hydrogel polymer into the gel-crushing apparatus and thus makes it possible to more smoothly carry out the gel-crushing step. Note that a means for chopping or breaking the hydrogel polymer is preferably a means that enables chopping or breaking of the hydrogel polymer without kneading the hydrogel polymer, and is, for example, a guillotine cutter or the like. The size and shape of the hydrogel polymer obtained by the chopping or breaking are not particularly limited, provided that the hydrogel polymer can be fed into the gel-crushing apparatus.

<Drying step>

The drying step is a step of drying the particulate hydrogel until a desired solid fraction is achieved, to obtain a particulate dried material. Examples of a drying method include, but are not particularly limited to, thermal drying, hot air drying, drying under reduced pressure, fluidized bed drying, infrared drying, microwave drying, drum dryer drying, drying by azeotropic dehydration with a hydrophobic organic solvent, and high humidity drying with use of high temperature water vapor. Note that a post-crosslinking agent (described later) may be used in the drying step to obtain a water absorbent resin powder that has been post-crosslinked (also referred to as surface-crosslinked) in the drying step.

[Dryer]

A dryer used in the drying step is not limited to any particular one, and one of or two or more of heat transfer dryers, heat radiating dryers, hot air heat transfer dryers, dielectric heating dryers, and the like are selected, as appropriate. The dryer may be of a batch type or of a continuous type. The dryer may be of a direct heating type or of an indirect heating type. The dryer may be any of material standing dryers, material stirring dyers, material transport dryers, and hot air transfer dryers. Examples of the dryer include heat transfer dryers such as dryers of a through-flow band type, a through-flow circuit type, a vertical through-flow type, a parallel flow band type, a through-flow tunnel type, a through-flow stirring type, a through-flow rotary type, a rotary type with a heating tube, a fluidized bed type, and an air flow type.

[Drying temperature]

A drying temperature in the drying step is 80°C or more, preferably 100°C or more, more preferably 120°C or more, and particularly preferably 150°C or more. The drying temperature is 250°C or less, preferably 230°C or less, and more preferably 220°C or less. Any combination is preferable for the upper limit and the lower limit of the drying temperature. The drying temperature of less than 80°C is not preferable, because a drying time to achieve a suitable resin solid content (moisture content) becomes longer. Furthermore, an undried material can generate and cause clogging during a subsequent pulverizing step. The drying temperature of more than 250°C is not preferable, because there are problems in safety and of generation of a colored foreign matter. In the case of direct heating, the drying temperature indicates the temperature of a heating medium used for drying. In the case of hot air drying, the drying temperature indicates the temperature of hot air used for drying. In the case of indirect heating, the drying temperature indicates the temperature of a heat transfer surface used for drying.

[Drying time]

A drying time in the drying step indicates a time period required for a solid content to become 80 weight% or more. The drying time is preferably 60 minutes or shorter, and preferably 40 minutes or shorter, 30 minutes or shorter, and 25 minutes or shorter in this order. The lower limit of the drying time is approximately 1 minute, in consideration of drying efficiency. Furthermore, a total drying time is preferably 120 minutes or shorter, and more preferably 100 minutes or shorter, 80 minutes or shorter, and 60 minutes or shorter in this order. In a case where the drying time is short, an undried material can generate and cause clogging during the subsequent pulverizing step.

[Resin solid content]

The particulate hydrogel obtained in the gel-crushing step is dried in the above-described drying step, so that the particulate hydrogel becomes a dried polymer. A resin solid content determined from a drying loss (measured after 1 g of a powder or particles are heated at 180°C for 3 hours) of the dried polymer is preferably 80 weight% or more, more preferably 85 weight% to 99 weight%, and even more preferably 86 weight% to 98 weight%.

<Post-crosslinking step>

This step is a step of adding, to the hydrogel after the polymerization and the dried material obtained therefrom, a post-crosslinking agent which reacts with a functional group (particularly, a carboxyl group) of a water absorbent resin, so as to cause a crosslinking reaction. Since crosslinking mainly occurs from surfaces of water absorbent resin particles, the crosslinking is also referred to as surface-crosslinking or secondary-crosslinking. As an example, in this step, a post-crosslinking agent is added to the particulate hydrogel and/or the particulate dried material so that a reaction is caused. This step includes a post-crosslinking agent adding step and a heat treatment step, and may include a cooling step after the heat treatment step, as necessary.

<Sizing step>

This step is a step of adjusting the particle size of the particulate dried material or the post-crosslinked particulate dried material. This sizing step makes it possible to obtain a water absorbent resin powder having a particle diameter or a particle size distribution which is more actively controlled.
Preferably, the sizing step includes a crushing step and/or a classification step. The crushing step is a step of crushing, with use of a crusher, the loosely agglomerating particulate dried material obtained through the drying step or the heat treatment step, so as to adjust the particle diameter. The classification step is a step of, with use of a classifier, removing coarse particles and a fine powder from the particulate dried material, the post-crosslinked particulate dried material, or the crushed material obtained therefrom. Ideal is the sizing step that allows a water absorbent resin powder, the particle diameter and the particle size distribution of which are controlled, to be obtained only by the crushing step. Depending on the particle diameter and the particle size distribution of a water absorbent resin powder, water absorption performance, handleability, and sense of use when the water absorbent resin powder is applied to hygienic materials, such as diapers and sanitary products, vary. Therefore, it is preferable to carry out the sizing step to obtain a water absorbent resin powder which has a desired particle diameter and a desired particle size distribution.

<Other steps>

In addition to the above-described steps, the method for producing a water absorbent resin powder may include a cooling step, an aqueous monomer solution preparing step, an additive adding step, a fine powder removing step, and a fine powder recycle step. The method may further include the other known steps.

Embodiment 2

The following description will discuss another embodiment of the present disclosure. For convenience, members having the same functions as members described in the above embodiment are given the same reference signs and description thereof will be omitted.

(Configuration of predicting system 1000a)

For example, a predicting apparatus 100 may measure a near-infrared absorption spectrum of an intermediate product at at least any one of the following points in time: before a polymerization step; between the polymerization step and a drying step; and after the drying step, which are included in a process for producing a water absorbent resin powder, and may output prediction information concerning a physical property of the intermediate product (or the produced resin powder) in any stage in the above process.

Moreover, in a method for producing a resin powder, a production condition of a water absorbent resin powder may be controlled in any one or more steps for producing the water absorbent resin powder, on the basis of prediction information outputted by the predicting apparatus 100.

Furthermore, in order to control the method for producing a resin powder, prediction information outputted by the predicting apparatus 100 may be used.

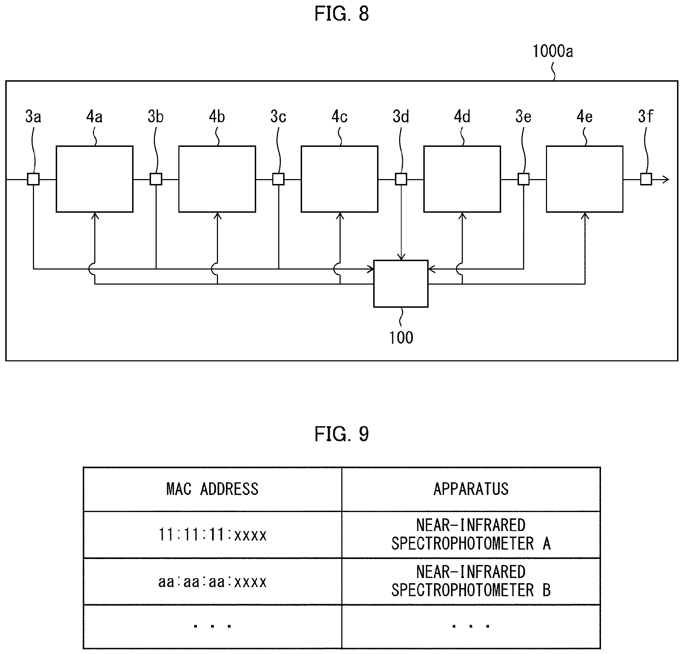

Furthermore, any production apparatus (corresponding to the external apparatus 4 illustrated in Fig. 1) which carries out any process step included in a process for producing a resin powder may be controlled on the basis of prediction information outputted from the predicting apparatus 100. A predicting system 1000a having such a configuration will be described with reference to Fig. 8. Fig. 8 is a block diagram illustrating an example of a configuration of the predicting system 1000a according to another embodiment of the present disclosure.

In Fig. 8, the predicting system 1000a includes a predicting apparatus 100, near-infrared spectrophotometers 3a to 3f, and external apparatuses 4a to 4e. The predicting apparatus 100 is connected to the near-infrared spectrophotometers 3a to 3f and the external apparatuses 4a to 4e. The external apparatuses 4a to 4e are each, for example, a control apparatus for carrying out a step (a polymerization step, a pulverizing step, or the like).

As an example, a case where the gel D50 of a water absorbent resin powder is predicted will be described below. The gel D50 is a physical property of the water absorbent resin powder which is measured after a gel-crushing step, for example. In Fig. 8, it is assumed that the external apparatus 4b is an apparatus which controls the gel-crushing step and the near-infrared spectrophotometer 3c is a near-infrared spectrophotometer which measures a near-infrared absorption spectrum after the gel-crushing step.

Firstly, the near-infrared spectrophotometer 3c outputs a measured near-infrared absorption spectrum of a water absorbent resin powder to the predicting apparatus 100. The predicting apparatus 100 carries out a preprocess(es) with respect to the obtained near-infrared absorption spectrum on the basis of a prediction model. Examples of the preprocess(es) include an outlier removing process and an averaging process. The predicting apparatus 100 predicts the gel D50 from the preprocessed near-infrared absorption spectrum on the basis of the prediction model. As an example, the predicting apparatus 100 outputs a prediction result to the external apparatus 4c. The external apparatus 4c may be, for example, an apparatus which controls a drying step.

For example, in a case where the predicting apparatus 100 predicts that a value of the gel D50 will be greater than a given value, the external apparatus 4c, which controls the drying step, may change a condition in the step, e.g., carry out control so that the resin powder is heated at a temperature higher than a given temperature.

In a case where the predicting apparatus 100 predicts that the value of the gel D50 will be greater than the given value, the external apparatus 4b, which controls the gel-crushing step, may increase a gel-crushing load so that the gel-crushing load is greater than a given load. Specifically, the external apparatus 4b may change a condition in the step, e.g., carry out control so that a rotation speed is increased and accordingly a higher shearing force is applied to a gel.

Another example is carrying out control so that the amount of a crosslinking agent in the polymerization step is reduced, in a case where the predicting apparatus 100 predicts that the CRC, which is a physical property of an end product, will be higher than CRC specified as a product standard. Another example is carrying out control so that the composition of an agent in a post-crosslinking step is changed, in a case where the AAP, which is a physical property of the end product, will be lower than AAP specified as a product standard.

In Embodiment 2, the predicting apparatus 100 may be able to identify which of the near-infrared spectrophotometers has obtained the near-infrared measurement data. As an example, the predicting apparatus 100 obtains, in advance, MAC addresses of the near-infrared spectrophotometers in the predicting system 1000a and places where the near-infrared spectrophotometers are disposed. As an example, Fig. 9 shows a table showing correspondence between the MAC addresses and the near-infrared spectrophotometers. In this manner, by also obtaining the MAC addresses of the near-infrared spectrophotometers in obtaining the near-infrared measurement data, the predicting apparatus 100 is able to identify in which step in the predicting system 1000a the near-infrared measurement data has been obtained.

The predicting system 1000a having such a configuration makes it possible to accurately predict, in a short time, a physical property of an intermediate product (e.g., a physical property, such as a gel particle diameter, of a pulverized gel) in each step for producing a resin powder or a physical property (e.g., the above-described CRC or AAP) of the resin powder which is an end product. By using prediction information to control any of production apparatuses (external apparatuses 4a to 4e) which each carry out a process in a process for producing the resin powder, it is possible to regulate, in real time, a physical property operational factor in each production process, and possible to effectively prevent production of an off-spec product.

The near-infrared spectrophotometers 3a to 3f are each generally inexpensive (at least less expensive than a Raman spectrophotometer). Thus, it is also possible to minimize a cost for disposing the near-infrared spectrophotometers 3a to 3f in the process for producing the resin powder.

[Software Implementation Example]

Control blocks (particularly, the control section 10) of the predicting apparatus 100 described in Embodiments 1 and 2 may be realized by a logic circuit (hardware) provided in an integrated circuit (IC chip) or the like or may be alternatively realized by software.

In the latter case, the predicting apparatus 100 includes a computer which executes instructions of a program that is software for realizing the foregoing functions. The computer includes, for example, at least one processor and a computer-readable storage medium in which the program is stored. An object of the present disclosure can be achieved by the processor of the computer reading the program from the storage medium and executing the program. Examples of the processor include a central processing unit (CPU). Examples of the storage medium include "non-transitory tangible media" such as read only memories (ROMs), tapes, disks, cards, semiconductor memories, and programmable logic circuits. The computer may further include a random access memory (RAM) or the like in which the program is loaded. Further, the program may be made available to the computer via any transmission medium (such as a communication network and a broadcast wave) which allows the program to be transmitted. Note that an aspect of the present disclosure can also be achieved in the form of a computer data signal in which the program is embodied via electronic transmission and which is embedded in a carrier wave.

The present disclosure is not limited to the embodiments above, and can be altered by a skilled person in the art within the scope of the claims. The present disclosure also encompasses, in its technical scope, any embodiment derived by combining, as appropriate, technical means disclosed in differing embodiments.

Examples

An example of the present disclosure will be described below. Note that, in order to adjust physical properties of samples to be subjected to near-infrared radiation absorption spectrum measurement, conditions of a polymerization step, a gel-crushing step, a drying step, a post-crosslinking step, a sizing step, and the other steps as described above were changed as appropriate, and then the samples to be subjected to the measurement was obtained. In the polymerization step (described later), the amount of polyethyleneglycol diacrylate, which was an internal crosslinking agent, was changed, for example. In the gel-crushing step (described later), the pore diameter of a porous plate was changed, for example. In the drying step (described later), a drying time was changed, for example. Moreover, in the post-crosslinking step (described later), the type of a post-crosslinking agent and the amount of the post-crosslinking agent used were changed, and, furthermore, a temperature during heat treatment and a time period of the heat treatment were changed, for example.

<Preparation of water absorbent resin (SAP)>

[Polymerization step]

In a polypropylene container having an inner diameter of 50 mm and a capacity of 120 mL, 23.2 g of acrylic acid, 0.135 g (0.080 mol%) of polyethyleneglycol diacrylate (having a weight average molecular weight [Mw] of 523 Da), 0.071 g of a 2.0 weight% aqueous diethylenetriamine pentaacetic acid trisodium solution, 22.2 g of ion-exchange water, and 9.6 g of a 48.5 weight% aqueous sodium hydroxide solution were mixed with each other to prepare a solution (A).

While the solution (A) was being stirred with a magnetic stirrer and adjusted at the temperature of 45°C, 9.8 g of a 48.5 weight% aqueous sodium hydroxide solution was added to the solution (A) over approximately 5 seconds and mixed with the solution (A) in an open system to prepare an aqueous monomer solution (1). Heat of neutralization and heat of dissolution caused during the mixing increased the temperature of the aqueous monomer solution (1) to approximately 80°C.

Subsequently, when the temperature of the aqueous monomer solution (1) reached 78°C, 1.01 g of a 4.5 weight% aqueous sodium persulfate solution was added, and a resulting mixture was stirred for approximately 3 seconds. Then, a resulting reaction liquid (1) was poured into a stainless-steel petri dish in an open system.

The stainless-steel petri dish had an inner diameter of 88 mm and a height of 20 mm. The stainless-steel petri dish had a surface heated in advance with use of a hot plate (NEO HOTPLATE H1-1000, manufactured by Iuchi Seiei Do Ltd.) so that the temperature thereof reached 50°C.

Immediately after the reaction liquid (1) was supplied, the stainless-steel petri dish was covered with a glass container having a discharge opening, and the inside air was sucked with use of a vacuum pump so that the pressure inside the casing was 85 kPa. The pressure outside the casing was 101.3 kPa (atmospheric pressure).

A while after the reaction liquid (1) was poured into the stainless-steel petri dish, polymerization started. As the polymerization proceeded, the reaction liquid (1) expanded and foamed upward in various directions while generating water vapor. Thereafter, a resulting polymer contracted to a size slightly larger than the bottom surface of the petri dish.

The expansion and contraction ended within approximately 1 minute. After being retained in the polymerization container (i.e., the stainless-steel petri dish covered with the glass container) for 3 minutes, the polymer, i.e., a crosslinked hydrogel polymer (hereinafter referred to as "hydrogel") (1), was then taken out.

[Gel-crushing step]

The obtained hydrogel (1) was gel-crushed with use of a screw extruder (meat chopper) having the following specifications. The screw extruder included a porous plate at an end thereof, and the porous plate had a diameter of 82 mm, a pore diameter of 8.0 mm, 33 pores, and a thickness of 9.5 mm. As conditions of the gel-crushing, the gel-crushing was carried out while the hydrogel (1) was being introduced in an amount of approximately 360 g/min and, simultaneously with the introduction of the hydrogel (1), deionized water at 90°C was being added at 50 g/min. A resulting gel-crushed particulate hydrogel (1) was used to evaluate gel D50 (described later).

[Drying step]

The gel-crushed particulate hydrogel (1) was spread onto a stainless-steel metal gauze having a mesh size of 850 pm, and dried with hot air at 190°C for 30 minutes. Subsequently, a dried polymer (1) obtained through this drying operation was pulverized with use of a roll mill (manufactured by Inoguchi Giken Ltd., WML-type roll crusher), and then a resulting pulverized dried polymer was classified with use of JIS standard sieves having respective mesh sizes of 710 pm and 175 pm to obtain a water absorbent resin powder (1).

[Post-crosslinking step]

A surface-crosslinking agent solution containing 0.025 g of ethylene glycol diglycidyl ether, 0.3 g of ethylene carbonate, 0.5 g of propylene glycol, and 2.0 g of deionized water was sprayed onto and mixed with 100 g of the water absorbent resin powder (1). A resulting mixture was subjected to heat treatment at 200°C for 35 minutes. Consequently, a surface-crosslinked water absorbent resin powder (2) was obtained.

Through the above series of operations, the water absorbent resin powders (1) and (2) each having a non-uniformly pulverized shape were obtained. The water absorbent resin powders were each used to evaluate CRC, AAP, SFC, D50, and a moisture content (solid content) (described later).

<Measurement of water absorbent resin>

Measurement apparatuses and measurement conditions for near-infrared absorption spectra were as below.

(i) Apparatus: FT-NIR NIRFlex (registered trademark) N-500 (manufactured by BUCHI)

Measurement wavelength: 800 nm to 2500 nm
Measurement method: diffuse reflection measurement

(ii) Apparatus: IRMA5184S (manufactured by Chino Corporation)

Measurement wavelengths (8 wavelengths): 1320 nm, 1460 nm, 1600 nm, 1720 nm, 1800 nm, 1960 nm, 2100 nm, and 2310 nm
Measurement method: near-infrared absorption type.

<Evaluation of performance of predicting apparatus for each physical property of water absorbent resin powder>

Wavelength data on obtained near-infrared absorption spectra was used as features. Physical property information on the samples subjected to the measurement was used as response variables. A relational expression between the features and the response variables was determined by a PCR method or a partial least squares (PLS) regression analysis method. The performance of a predicting apparatus was evaluated in terms of the following physical properties of the water absorbent resin powders: (1) gel D50; (2) CRC; (3) AAP; (4) SFC; (5) D50; and (6) a solid content.

Datasets used for the evaluation each included a plurality of combinations of near-infrared measurement data and a physical property which was associated with the near-infrared measurement data. The datasets were each divided into training data and validation data for use in the evaluation. Note, here, that the training data was data which contained near-infrared absorption spectra and actually measured values of the physical property which were associated with the respective near-infrared absorption spectra, and was data for use in prior machine learning. Note also that the validation data was data which was not included in the training data. In the present example, the datasets used for the evaluation were each randomly divided, and a prediction model was generated by carrying out PLS or PCR with respect to the training data.

Firstly, near-infrared absorption spectra and physical properties of a plurality of water-absorbing resin powders (1) and (2) were measured. Then, datasets were prepared which each contained N combinations of near-infrared absorption spectra and a physical property which was associated with the near-infrared absorption spectra. The datasets were each divided into training data and validation data such that 80% of the combinations were used for training and 20% of the combinations were used for validation.

Graphs obtained by making plotting in regard to the respective physical properties are shown below. In each graph, data indicated by "training" is training data, and data indicated by "test" is validation data. A dotted line shown in each graph indicates a true regression line obtained when actually measured values of a physical property and predicted values of the physical property completely match each other. Predicted values of each physical property versus actually measured values of the physical property in the training data and the validation data were plotted. As points shown by plotting the actually measured values and the predicted values are closer to the regression line in each graph, it can be determined that the predicting apparatus has higher performance.

(1) Gel D50

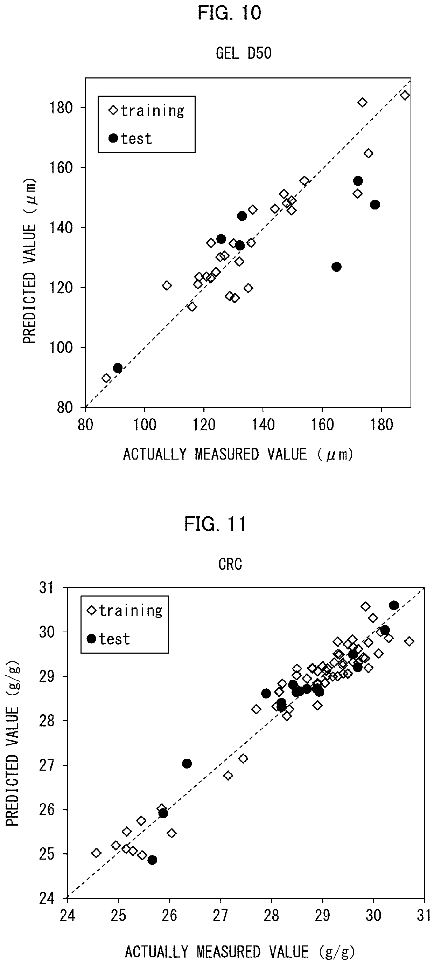

A dataset had 36 combinations of near-infrared measurement absorption spectra and a physical property which was associated with the near-infrared absorption spectra. The dataset was randomly divided into training data and validation data, and 80% were used for training and 20% were used for validation. PCR was carried out with respect to the training data to generate a prediction model. Fig. 10 is a graph obtained by plotting, in a range of 80 $\mu$m to 190 pm, predicted values of gel D50 versus actually measured values of the gel D50.

(2) CRC

A dataset had 79 combinations of near-infrared measurement absorption spectra and a physical property which was associated with the near-infrared absorption spectra. The dataset was randomly divided into training data and validation data, and 80% were used for training and 20% were used for validation. PLS was carried out with respect to the training data to generate a prediction model. Fig. 11 is a graph obtained by plotting, in a range of 24 g/g to 31 g/g, predicted values of CRC versus actually measured values of the CRC.

(3) AAP

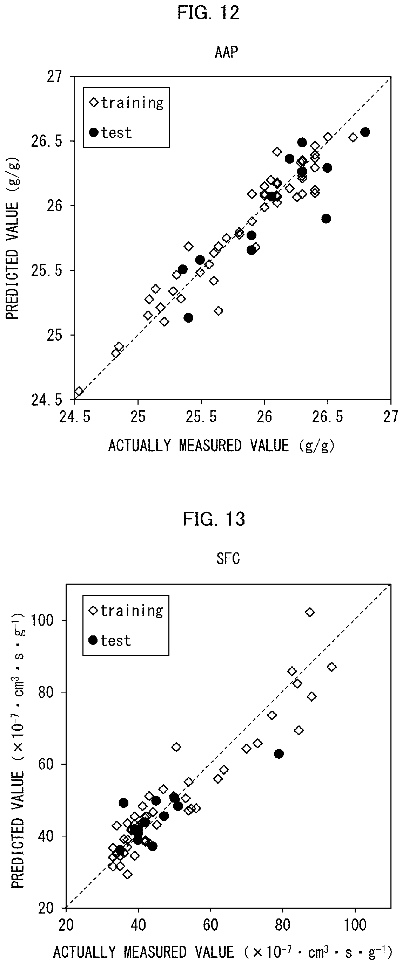

A dataset had 69 combinations of near-infrared measurement absorption spectra and a physical property which was associated with the near-infrared absorption spectra. The dataset was randomly divided into training data and validation data, and 80% were used for training and 20% were used for validation. PLS was carried out with respect to the training data to generate a prediction model. Fig. 12 is a graph obtained by plotting, in a range of 24.5 g/g to 27 g/g, predicted values of AAP versus actually measured values of the AAP.

(4) SFC

A dataset had 64 combinations of near-infrared measurement absorption spectra and a physical property which was associated with the near-infrared absorption spectra. The dataset was randomly divided into training data and validation data, and 80% were used for training and 20% were used for validation. PLS was carried out with respect to the training data to generate a prediction model. Fig. 13 is a graph obtained by plotting, in a range of 20 to 110 ($\times 10^{-7} \cdot cm^3 \cdot s \cdot g^{-1}$), predicted values of SFC versus actually measured values of the SFC.

(5) D50

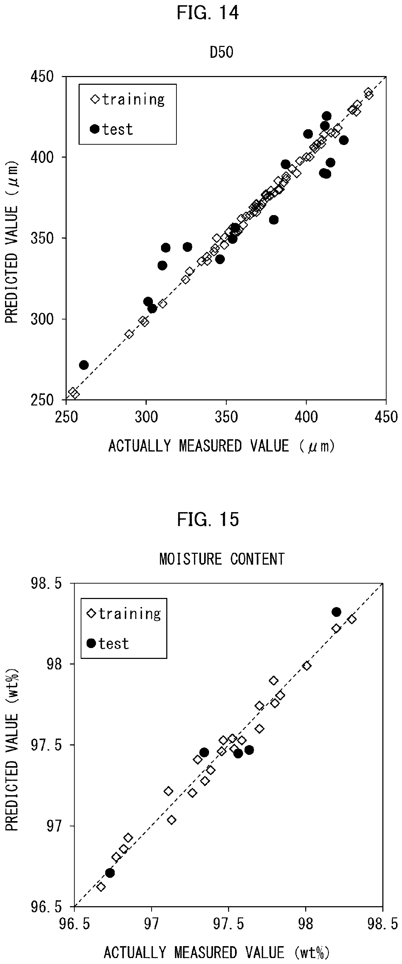

A dataset had 90 combinations of near-infrared measurement absorption spectra and a physical property which was associated with the near-infrared absorption spectra. The dataset was randomly divided into training data and validation data, and 80% were used for training and 20% were used for validation. PLS was carried out with respect to the training data to generate a prediction model. Fig. 14 is a graph obtained by plotting, in a range of 250 pm to 450 pm, predicted values of D50 versus actually measured values of the D50.

(6) Moisture content (solid fraction)

A dataset had 29 combinations of near-infrared measurement absorption spectra and a physical property which was associated with the near-infrared absorption spectra. The dataset was randomly divided into training data and validation data, and 80% were used for training and 20% were used for validation. PLS was carried out with respect to the training data to generate a prediction model. Fig. 15 is a graph obtained by plotting, in a range of 96.5 wt% to 98.5 wt%, predicted values of a moisture content versus actually measured values of the moisture content. A solid content is determined by 100-the moisture content (weight%), and therefore the graph also shows predicted values of the solid fraction versus actually measured values of the solid fraction.

<Evaluation result>

With regard to all of the physical properties, the predicted values correlated well with the actually measured values. Also with regard to the validation data which was not included in the training data, it was possible to predict the physical properties with accuracy similar to that for the training data. Thus, it was shown that the predicting apparatus 100 had good performance.

Reference Signs List

100  Predicting apparatus
11   Measurement data obtaining section
13   Predicting section
22   Prediction model
23   Physical property information

**Claims**

1.  A method for predicting a physical property of a resin powder,

    the resin powder being any one of a water absorbent resin powder and an intermediate product which is produced in a process for producing the water absorbent resin powder,
    said method comprising:

    a near-infrared measurement data obtaining step of obtaining near-infrared measurement data which indicates a near-infrared absorption spectrum of the resin powder; and
    a predicting step of inputting, into a prediction model, at least one selected from the group consisting of the near-infrared measurement data and one or more pieces of processed data which have been generated on the basis of the near-infrared measurement data, and outputting prediction information concerning the physical property of the resin powder.

2.  The method according to claim 1, wherein the prediction model is a prediction model which has been generated by machine learning in which at least any one of the following (1) and (2) is used as training data: (1) a combination of near-infrared measurement data and physical property information, the near-infrared measurement data containing near-infrared absorption spectra of a plurality of produced resin powders which have been previously produced and have each known physical property, the physical property information being on end products each of which is associated with the near-infrared measurement data; and (2) a combination of near-infrared measurement data and physical property information, the near-infrared measurement data containing near-infrared absorption spectra of a plurality of produced intermediate products which have been produced in a process for producing a corresponding one of the plurality of produced resin powders and have each known physical property, the physical property information being on the plurality of produced intermediate products each of which is associated with the near-infrared measurement data.

3.  The method according to claim 2, wherein the prediction model is generated with use of any one of linear regression and non-linear regression.

**4.** The method according to claim 2 or 3, wherein the prediction model is generated with use of any one of principal component regression and partial least squares regression.

**5.** The method according to any one of claims 1 to 4, further comprising:

a preprocessing step of generating the one or more pieces of processed data,
in the preprocessing step, any one or more of an outlier removal process, an averaging process, a wavelength range selection process, and a differential process being carried out.

**6.** The method according to any one of claims 1 to 5, wherein the prediction information includes at least any one of (1) a mass average particle diameter (gel D50) of a hydrogel which is the intermediate product, (2) an absorption capacity without load (CRC) of the resin powder, (3) an absorption capacity under load (AAP) of the resin powder, (4) a saline flow conductivity (SFC) of the resin powder, (5) a mass average particle diameter (D50) of the resin powder, and (6) an amount of a solid component contained in the resin powder or a solid fraction of the resin powder.

**7.** The method according to any one of claims 1 to 6, wherein:

the process for producing the resin powder includes a polymerization step and a drying step;
the near-infrared absorption spectrum is measured at at least any one of the following points in time: before the polymerization step; between the polymerization step and the drying step; and after the drying step; and
any one or more production apparatuses which are used in the process for producing the resin powder are controlled on the basis of the prediction information which has been outputted in the predicting step.

**8.** A predicting apparatus which predicts a physical property of a resin powder,

the resin powder being any one of a water absorbent resin powder and an intermediate product which is produced in a process for producing the water absorbent resin powder,
said predicting apparatus comprising:

a measurement data obtaining section which obtains near-infrared measurement data that indicates a near-infrared absorption spectrum measured with respect to the resin powder; and
a predicting section which inputs, into a prediction model, at least any one selected from the group consisting of the near-infrared measurement data and one or more pieces of processed data which have been generated on the basis of the near-infrared measurement data, and outputs prediction information concerning the physical property of the resin powder.

**9.** A method for producing a resin powder which comprises a polymerization step and a drying step, on the basis of prediction information obtained by the method recited in any one of claims 1 to 7, a production condition of the resin powder being controlled in any one or more steps for producing the resin powder.

**10.** Use of prediction information which has been obtained by the method recited in any one of claims 1 to 7, for controlling a method for producing a resin powder.

**11.** A method for measuring a near-infrared absorption spectrum of a resin powder, the near-infrared absorption spectrum being used in the method recited in any one of claims 1 to 6, said method comprising:

a step of irradiating the resin powder with near-infrared radiation; and
a step of calculating the near-infrared absorption spectrum of the resin powder from a measurement value obtained by measuring at least one of light reflected by the resin powder and light transmitted by the resin powder, the resin powder being any one of a water absorbent resin powder and an intermediate product which is produced in a process for producing the water absorbent resin powder.

## FIG. 1

NEAR-INFRARED
SPECTROPHOTOMETER `3`

`1000`

MEASUREMENT DATA

`100`
PREDICTING APPARATUS

CPU `1`

`2`

MEMORY

PREDICTION INFORMATION

EXTERNAL APPARATUS `4`

## FIG. 2

NEAR-INFRARED
SPECTROPHOTOMETER  /3

MEASUREMENT DATA

PREDICTING APPARATUS  /100

CONTROL SECTION  /10

MEASUREMENT DATA
OBTAINING SECTION  /11

PREDICTING SECTION  /13

STORAGE SECTION  /20

PREDICTION MODEL  /22

COMMUNICATION
SECTION  /50

PREDICTION INFORMATION

EXTERNAL APPARATUS  /4

FIG. 3

```
        ┌──────────────────────────────┐
        │      PREDICTING PROCESS       │
        └──────────────────────────────┘
                       │
   ┌───────────────────────────────────────┐
   │  OBTAIN NEAR-INFRARED MEASUREMENT DATA │ ⌐S1
   └───────────────────────────────────────┘
                       │
   ┌───────────────────────────────────────┐
   │ READ OUT PREDICTION MODEL FROM STORAGE SECTION │ ⌐S2
   └───────────────────────────────────────┘
                       │
   ┌───────────────────────────────────────┐
   │  INPUT NEAR-INFRARED MEASUREMENT DATA INTO │ ⌐S3
   │           PREDICTION MODEL             │
   └───────────────────────────────────────┘
                       │
   ┌───────────────────────────────────────┐
   │        PREDICT PHYSICAL PROPERTY       │ ⌐S4
   └───────────────────────────────────────┘
                       │
   ┌───────────────────────────────────────┐
   │        OUTPUT PREDICTION RESULT        │ ⌐S5
   └───────────────────────────────────────┘
                       │
                ┌─────────────┐
                │     END      │
                └─────────────┘
```

## FIG. 4

┌─────────────────────────────────────────────────────────────────────────┐
│                          ┌ 100                                           │
│                     PREDICTING APPARATUS                                  │

┌──────────────────────────────────┐      ┌─────────────────────────────┐
│         ┌ 10                      │      │       ┌ 20                   │
│      CONTROL SECTION              │      │   STORAGE SECTION           │
│                                   │      │              ┌ 21           │
│   ┌ 11                            │      │    ┌─────────────────────┐   │
│ ┌──────────────────┐             │◄─────┼────│  NEAR-INFRARED      │   │
│ │ MEASUREMENT DATA │             │      │    │ MEASUREMENT DATA    │   │
│ │ OBTAINING SECTION│             │      │    └─────────────────────┘   │

(The figure shows the Predicting Apparatus 100 containing a Control Section 10 with a Measurement Data Obtaining Section 11, a Predicting Section 13, and a Prediction Model Generating Section 18, and a Storage Section 20 containing Near-Infrared Measurement Data 21, Physical Property Information 23, and Prediction Model 22.)

- CONTROL SECTION 10
  - MEASUREMENT DATA OBTAINING SECTION 11
  - PREDICTING SECTION 13
  - PREDICTION MODEL GENERATING SECTION 18
- STORAGE SECTION 20
  - NEAR-INFRARED MEASUREMENT DATA 21
  - PHYSICAL PROPERTY INFORMATION 23
  - PREDICTION MODEL 22

## FIG. 5

┌ 21

| MEASUREMENT ID | NEAR-INFRARED ABSORPTION SPECTRUM |
|----------------|-----------------------------------|
| 001 | 📁 |
| 002 | 📁 |
| . . . | . . . |

## FIG. 6

| MEASUREMENT ID | PHYSICAL PROPERTY (ACTUALLY MEASURED VALUE) |
|----------------|----------------------------------------------|
| 001 | |
| 002 | |
| . . . | . . . |

## FIG. 7

GENERATION OF PREDICTION MODEL

READ OUT NEAR-INFRARED ABSORPTION SPECTRUM GROUP AND PHYSICAL PROPERTY GROUP FROM STORAGE SECTION — S11

GENERATE OR UPDATE PREDICTION MODEL CANDIDATE — S12

INPUT NEAR-INFRARED ABSORPTION SPECTRUM GROUP INTO PREDICTION MODEL CANDIDATE — S13

OUTPUT PREDICTION RESULT OF PREDICTING PHYSICAL PROPERTY GROUP CORRESPONDING TO INPUTTED NEAR-INFRARED ABSORPTION SPECTRUM GROUP — S14

COMPARE PHYSICAL PROPERTY GROUP WHICH IS ASSOCIATED WITH INPUTTED NEAR-INFRARED ABSORPTION SPECTRUM GROUP WITH PREDICTION RESULT, AND CALCULATE MODEL EVALUATION INDEX — S15

DOES PREDICTION MODEL CANDIDATE SATISFY EVALUATION CRITERION? — S16

NO

YES

STORE OPTIMAL PREDICTION MODEL CANDIDATE AS PREDICTION MODEL — S17

END

FIG. 8

1000a

FIG. 9

| MAC ADDRESS | APPARATUS |
|---|---|
| 11:11:11:xxxx | NEAR-INFRARED SPECTROPHOTOMETER A |
| aa:aa:aa:xxxx | NEAR-INFRARED SPECTROPHOTOMETER B |
| . . . | . . . |

## FIG. 10

### GEL D50

## FIG. 11

### CRC

FIG. 12

AAP

FIG. 13

SFC

## FIG. 14

D50

## FIG. 15

MOISTURE CONTENT

## INTERNATIONAL SEARCH REPORT

| International application No. |
|---|
| **PCT/JP2021/037735** |

**A.  CLASSIFICATION OF SUBJECT MATTER**

*G01N 21/359*(2014.01)i
FI:   G01N21/359

According to International Patent Classification (IPC) or to both national classification and IPC

**B.  FIELDS SEARCHED**

Minimum documentation searched (classification system followed by classification symbols)

G01N 21/00-21/01; G01N 21/17-21/61; G01J 3/00-3/51; G01N 15/00-15/14; G01N 33/00; G01N 33/44; B01J 20/22-20/26; C08J 3/00-3/28; G06N 3/00-3/12; G06N 20/00-20/20; G16Z 99/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
|---|---|---|
| Y | JP 09-268232 A (NIPPON SHOKUBAI CO LTD) 14 October 1997 (1997-10-14) paragraphs [0071]-[0074], fig. 16-17 | 1–11 |
| Y | JP 2002-090298 A (SPECTRON TECH CO LTD) 27 March 2002 (2002-03-27) paragraphs [0033]-[0044], fig. 3-7 | 1–11 |
| Y | JP 2003-344279 A (SHISEIDO CO LTD) 03 December 2003 (2003-12-03) paragraphs [0025]-[0045], fig. 2-4 | 1–11 |
| Y | WO 2012/043821 A1 (NIPPON SHOKUBAI CO LTD) 05 April 2012 (2012-04-05) paragraphs [0092]-[0095] | 7, 9-10 |
| A | JP 2013-018917 A (KAO CORP) 31 January 2013 (2013-01-31) paragraphs [0043]-[0052] | 1–11 |

☑ Further documents are listed in the continuation of Box C.       ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **19 December 2021** | **28 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2021/037735**

C. DOCUMENTS CONSIDERED TO BE RELEVANT

| Category* | Citation of document, with indication, where appropriate, of the relevant passages | Relevant to claim No. |
|---|---|---|
| A | 松田　公昭　他, ポリアクリロニトリルのアンモニア加水分解吸水性樹脂とその吸水特性, 高分子学会予稿集, 12 September 1989, vol. 38, pp. 3925-3927<br>in particular, pp. 3926-3927, fig. 6, (MATSUDA, Masaaki et al. Hydrolysis of polyacrylonitrile by ammonium hydroxide; water absorbing performance of the hydrolyzed polymer. Polymer Preprints, Japan.) | 1–11 |

Form PCT/ISA/210 (second sheet) (January 2015)

## EP 4 231 000 A1

**INTERNATIONAL SEARCH REPORT**
Information on patent family members

| International application No. |
|---|
| **PCT/JP2021/037735** |

| Patent document cited in search report | | | Publication date (day/month/year) | Patent family member(s) | | | Publication date (day/month/year) |
|---|---|---|---|---|---|---|---|
| JP | 09-268232 | A | 14 October 1997 | WO 97/28209 A1 US 6150469 A column 29, lines 14-39, fig. 17-18 EP 819721 A1 | | | |
| JP | 2002-090298 | A | 27 March 2002 | US 6529767 B1 column 5, line 16 to column 6, line 47, fig. 3-7 EP 1184663 A2 | | | |
| JP | 2003-344279 | A | 03 December 2003 | (Family: none) | | | |
| WO | 2012/043821 | A1 | 05 April 2012 | US 2013/0175473 A1 paragraphs [0152]-[0154] EP 2623198 A1 | | | |
| JP | 2013-018917 | A | 31 January 2013 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020109601 A
- WO 2016204302 A
- US 5669894 A
- US 20120318046 A
- US 7638570 B

- WO 2009016055 A
- US 7265190 B
- JP 2000063527 A
- WO 2011126079 A

**Non-patent literature cited in the description**

- Non-Woven Standard Procedures. 2015